# EUROPEAN PATENT SPECIFICATION

(11) **EP 0 389 110 B1**
(45) Date of publication and mention of the grant of the patent: **03.05.1995**
(21) Application number: 90301928.9
(22) Date of filing: 22.02.1990
(51) Int. Cl.: C07H 19/073

(54) **Process for the preparation of 2'-deoxy-5-trifluoromethyl-beta-uridine**
Verfahren zur Herstellung von 2'Deoxy-5-trifluoromethyl-ss-uridin
Procédé pour préparer la 2'-déoxy-5-trifluorméthyl-ss-uridine

(30) Priority: 22.02.1989 JP 40156/89; 18.01.1990 JP 7234/90
(43) Date of publication of application: 26.09.1990
(73) Proprietor: YUKI GOSEI KOGYO CO., LTD., Chiyoda-ku Tokyo 102 (JP); Japan Tobacco Inc., Minato-Ku Tokyo 105 (JP)
(72) Inventor: Itoh, Kazuo, Tokyo Laboratory, Itabashi-ku, Tokyo 174 (JP); Naoi, Yoshitake, Tokyo Laboratory, Itabashi-ku, Tokyo 174 (JP); Matsushita, Hajime Life Science Research Lab., Yokohama-shi kanagawa 227 (JP); Ebata, Takashi, Life Science Research Laboratory, Yokohama-shi, Kanagawa 227 (JP); Kawakami, Hiroshi Life Science Research Lab., Yokohama-shi Kanagawa 227 (JP)
(74) Representative: Holmes, Michael John

(56) References cited:
- EP-A- 0 300 765
- EP-A- 0 350 293
- DE-A- 1 919 307
- DE-A- 1 943 428
- US-A- 3 721 664

## Description

### 1. Background of the Invention

### (1) Field of the Invention

The present invention relates to a process for preparing 2′-deoxy-5-trifluoromethyl-β-uridine which is useful per se as a carcinostatic agent and is a raw material for other pharmaceutically useful compounds.

### (2) Description of the Prior Art

2′-deoxy-5-trifluoromethyl-β-uridine is useful per se as a carcinostatic agent and is a raw material for other pharmaceutically useful compounds, and thus the demand for it is increasing.

In the conventional process for preparing 2′-deoxy-5-trifluoromethyl-β-uridine, the following methods are known:
(a) a method for transforming the nucleic base from thymidine and 5-trifluoromethyluracil with an enzyme such as nucleoside-2′-deoxyribose transferase [Methods Carbohydr. Chem., 7, 19 (1976)];
(b) a method of chemical synthesis by preparing an anomeric mixture by the condensation of a 5-trifluoromethyluracil derivative and a 2-deoxy-D-erythro-pentofuranose derivative in the presence of a catalyst and isolating the β-isomer as the desired product [J. Org. Chem., 31, 1181 (1966); USP 3531464]; and
(c) a method of halogenating the 5-position of 2′-deoxy-β-uridine as a raw material and then subjecting the halogenated product to trifluoromethylation [J. Chem. Soc. Perkin Trans. I, 2755, 1980].

In addition, EP-A-300765 describes a process for preparing pyrimidine 2-deoxynucleosides using cuprous iodide as a catalyst in the reaction of pyrimidines with 2-deoxy sugars.

However, the aforementioned production methods have the following drawbacks:
The production method (a) is not suited for a large scale production because of difficulties in the isolation of the aimed product or the productivity; a harmful mercury salt is used and complicated and time-consuming operations are needed for isolating the β-isomer as the aimed product from the anomeric mixture in the production method (b); and 2'-deoxy-β-uridine as a raw material is expensive in the production method (c).

Thus, none of the methods (a)-(c) are suitable for industrial production.

Therefore, a process for the preparation of 2'-deoxy-5-trifluoromethyl-β-uridine suitable for industrial application has been desired whereby the aforementioned problems of conventional methods are resolved and a large amount of the product can be easily produced.

### 2. Summary of the Invention

We have the conducted research into a process for the selective and efficient preparation of the β-isomer, represented by the following formula (I), without the problems described above. As a result, we have found that the condensation reaction of compound (III) and compound (IV) proceeds with a selectivity of 90% or more to the β-isomer and with a high yield, and after the product is purified, the deprotection reaction of the thus purified compound (II) gives compound (I).

Hence, the present invention is a process for the preparation of 2'-deoxy-5-trifluoromethyl-β-uridine represented by formula (I) 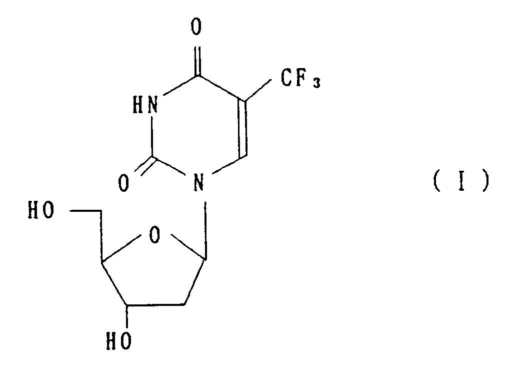
characterized in that a 5-trifluoromethyl-2,4-bis(triorganosilyloxy)pyrimidine represented by the general formula (IV) 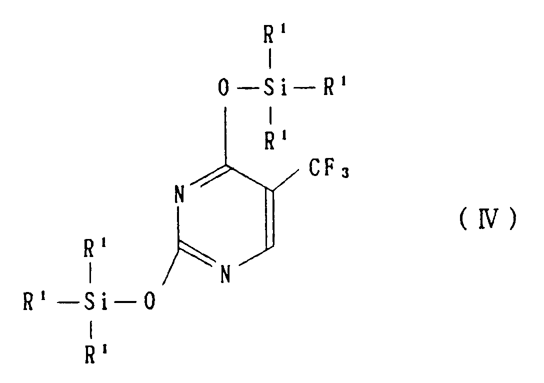
(wherein each R', which may be the same or different, represents an alkyl group which may be substituted,or a phenyl group)
and a 1-halogeno-2-deoxy-α-D-erythro-pentofuranose derivative represented by the general formula (III) 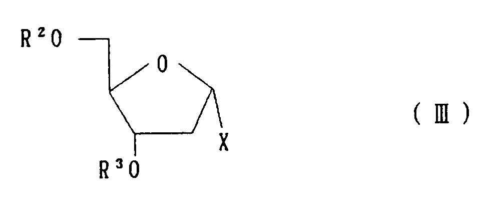
(wherein R² and R³ represent a general protective group for a hydroxyl group, and X represents a halogen atom)
are subjected to a condensation reaction to give a 1-(2-deoxy-β-D-erythro-pentofuranosyl)-5-trifluoromethyluracil derivative represented by the general formula (II) 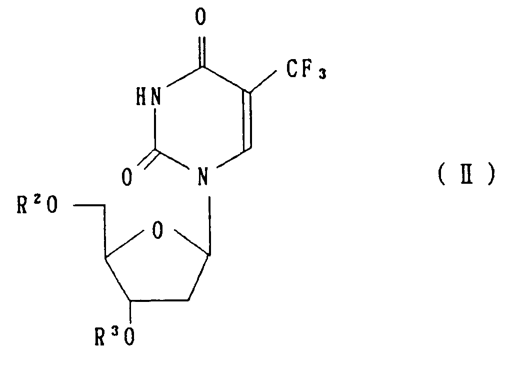
(wherein R² and R³ represent a general protective group for a hydroxyl group)
which is then subjected to a deprotection reaction to give 2'-deoxy-5-trifluoromethyl-β-uridine.

### 4. Description of the Preferred Embodiments

The compound (IV), one of the raw materials of the present invention is preferably obtained by the silylation of 5-trifluoromethyluracil as described in J. Org. Chem., 31, 1181 (1966) and USP 3531464.

5-trifluoromethyluracil is commercially available and is described by Heidenberger et al. [J. Med. Chem., 7, 1 (1964)] or by Fuchigami et al. [Tetrahedron Lett., 23, 4099 (1982)].

The triorganosilyl group of compound (IV) includes, for example, general protective groups for a hydroxyl group such as a trimethylsilyl group, a t-butyldimethylsilyl group, a phenyldimethylsilyl group or the like without limit thereto.

The compound (III), another raw material of the present invention, is prepared, for example, by the method described by Fox et al. [J. Am. Chem. Soc., 83, 4066 (1961)].

The protective groups of the two hydroxyl groups in the compound (III) include those which are generally used as a protective group of saccharides, for example, aralkyl groups such as benzyl, trityl and the like; acyl groups such as acetyl, propionyl, pivaloyl, benzoyl and the like; alkyloxycarbonyl groups such as ethoxycarbonyl and the like; and aryloxycarbonyl groups such as phenoxycarbonyl and the like without limit thereto. When the protective groups have a phenyl group, they may have as a substituent thereof an alkyl group, a halogen atom, a nitro group, an alkoxy group or the like.

The condensation reaction of compound (IV) and compound (III) is performed in an appropriate solvent, preferably in halogen containing hydrocarbons, more preferably in chloroform, at an ambient temperature and is completed generally within 15 hours.

The condensation reaction can be carried out in the presence of a catalyst such as a Lewis acid (e.g., 15% by mole of zinc chloride) or in the absence of the Lewis acid catalyst.

The molar ratio of compound (III) and compound (IV) in the condensation reaction is conveniently in the range of 1 : 1 to 1 : 10, preferably 1 : 4.

The reaction mixture thus obtained is crystallized to give the purified β-isomer of the compound (II).

The product is then subjected to deprotection by hydrolysis, alkanolysis,ammonolysis or the like to give 2′-deoxy-5-trifluoromethyl-β-uridine.

### Examples

The present invention will be ilustrated by reference to the following non-limiting examples.

In this connection, for all of the examples, 5-trifluoromethyl-2,4-bis(trimethylsilyloxy)pyrimidine was obtained by the silylation of a commercially available 5-trifluoromethyluracil, and 1-chloro-2-deoxy-3,5-di-O-(p-chlorobenzoyl)-α-D-erythro-pentofuranose was obtained by the method described by the aforementioned Fox et al. [J. Am. Chem. Soc., 83, 4066 (1961)].

### Example 1

### Synthesis of 1-[2-deoxy-3,5-di-O-(p-chlorobenzoyl)-β-D-erythro-pentofuranosyl]-5-trifluoromethyluracil

To a solution of 3.89 g (12.0 mmol) of 5-trifluoromethyl-2,4-bis(trimethylsilyloxy)pyrimidine in 24 ml of chloroform was added 60 mg (0.44 mmol ) of zinc chloride as a Lewis acid catalyst under dry conditions. Next, 1.25 g (2.91 mmol ) of 1-chloro-2-deoxy-3,5-di-O-(p-chlorobenzoyl)-α-D-erythropentofuranose in the form of dry powder was added to the mixture, and the reaction mixture was stirred at an ambient temperature for 15 hours.

After the reaction was completed, the reaction solution was poured into an aqueous sodium hydrogen carbonate solution and extracted with chloroform. The organic layer was dried over anhydrous magnesium sulfate, and the solvent was evaporated under reduced pressure.

The residue thus obtained was crystallized from ethanol to give 1.34 g of the title compound (yield 80%).
Melting point : 173 - 176°C;
¹H-NMR(CDCℓ₃): δ 8.05(s,1H,H-6),
7.99(d,J=8.7Hz,1H,aromatic H),
7.92(d,J=8.7Hz,1H,aromatic H),
7.46(d,J=8.7Hz,1H,aromatic H),
7.43(d,J=8.7Hz,1H,aromatic H),
6.30(dd,J=8.3&5.5Hz,1H,H-1′ ),
5.61(d,J=6.6Hz,1H,H-3 ′ ),
4.74(d,J=3.7Hz,2H,H-5 ′ ),
4.61(dd,J=5.9&3.6Hz,1H,H-4′ ),
2.88(ddd,J=14.5&5.6&1.6Hz,1H,H-2 ′ ),
2.31(ddd,J=14.7&8.0&6.7Hz,1H,H-2 ˝ )

### Synthesis of 2′-deoxy-5-trifluoromethyl-β-uridine

To a solution of 1.32 g (2.30 mmol ) of 1-[2-deoxy-3,5-di-O-(p-chlorobenzoyl)-β-D-erythro-pentofuranosyl]-5-trifluoromethyluracil in 80 ml of methanol was added 0.16 g (3.0 mmol ) of sodium methoxide, and the mixture was stirred at an ambient temperature for 1 hour.

After completing the reaction, a pyridinium type cation exchange resin was added to the reaction solution, and stirring was conducted for 10 minutes until the solution was neutralised.

After the resin was removed by filtration, the solvent was concentrated under reduced pressure, and the resulting residue was dissolved in water and washed twice with chloroform. The residue obtained by concentrating the aqueous layer under reduced pressure was purified by silica gel column chromatography (chloroform : methanol = 85 : 15), and the resulting crystals were subjected to recrystallization from ethanol to give 458 mg of the title compound (yield 67 %).
Melting point : 177 - 179°C (lit. : 186 - 189°C)
¹H-NMR(CD₃OD) : δ 8.81(s,1H,H-6),
6.24(t,J= 6.2Hz,1H,H-1′ ),
4,41(dt,J=5.9&4.1Hz,1H,H-3′ ),
3.96(dd,J=6.4&2.9Hz,1H,H-4′ ),
3.84(dd,J= 11.9&2.8Hz,1H,H-5 ′ ),
3.74(dd,J=11.9&2.8Hz,1H,H-5˝ ),
2.37(ddd,J=13.7&6.3&4.4Hz,1H,H-2 ′ ),
2.27(dq,J=13.1&6.2Hz,1H,H-2˝ )

### Example 2

### Synthesis of 1-[2-deoxy-3,5-di-O-(p-chlorobenzoyl)-β-D-erythro-pentofuranosyl]-5-trifluoromethyluracil

To a solution of 12.9 kg (39.7 mol ) of 5-trifluoromethyl-2,4-bis(trimethylsilyloxy)pyrimidine in 210 litres of chloroform was added 32.9 g (0.24 mmol ) of zinc chloride under dry conditions. Next, 10.5 kg (24.4 mol ) of 1-chloro-2-deoxy-3,5-di-O-(p-chlorobenzoyl)-α-D-erythro-pentofuranose in the form of dry powder was added to the mixture, and the reaction mixture was stirred at an ambient temperature for 5.5 hours.

After the reaction was completed, the reaction solution was treated in the same manner as in Example 1 to give 10.4 kg of the title compound (yield 74%).

### Example 3

### Condensation reaction in the absence of a Lewis acid catalyst

Under dry conditions, 2.5 mmol of 5-trifluoromethyl-2,4-bis(trimethylsilyloxy)pyrimidine as compound (IV) was added to 7.5 ml of chloroform. Then, a suspension of a predetermined amount of 1-chloro-2-deoxy-3,5-di-O-(p-chlorobenzoyl)-α-D-erythro-pentofuranose as compound (III) in the form of dry powder in 5 ml of chloroform was added with stirring to the mixture, and the reaction mixture was stirred at an ambient temperature for about 20 hours.

The amount of compound (III) was adjusted depending on the molar concentration (0.2 mol /litre) of compound (IV) to ensure that the molar ratio of compound (III) : compound (IV) is in the range of 1 : 1 to 1 : 4.

Then, the reactions (Nos. 1 - 4) were carried out with various amounts of compound (III).

After the reaction was completed, the reaction solution was subjected to HPLC determination to obtain the analytical yield of the α-isomer and β-isomer and the selectivity represented by the molar ratio of α-isomer : β-isomer.

Results are shown in the following table.

**Table**

| | Molar ratio (III) : (IV) | Analytical yield (%) | Selectivity α-isomer : β-isomer |
|---|---|---|---|
| No. 1 | 1 : 1 | 100 | 10 : 90 |
| No. 2 | 1 : 2 | 100 | 7 : 93 |
| No. 3 | 1 : 3 | 100 | 4 : 96 |
| No. 4 | 1 : 4 | 100 | 3 : 97 |

## Claims

1. A process for the preparation of 2'-deoxy-5-trifluoromethyl-β-uridine represented by formula (I) 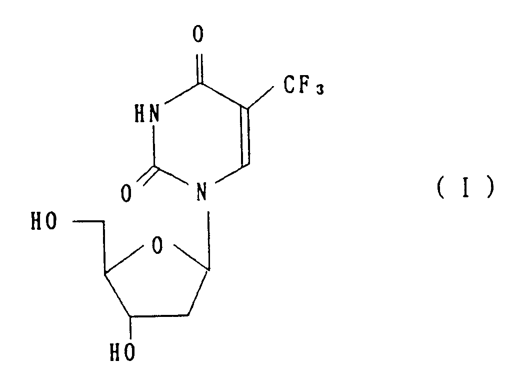 characterized in that a 5-trifluoromethyl-2,4-bis(triorganosilyloxy)pyrimidine represented by the general formula (IV) 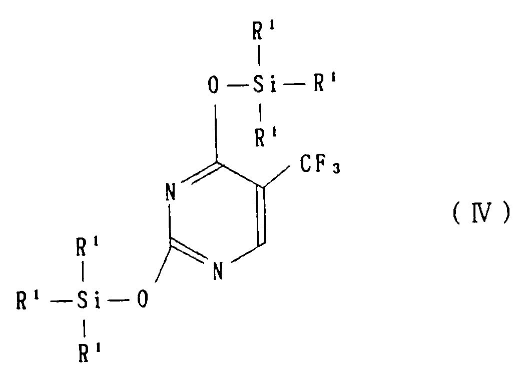 (wherein each R', which may be the same or different, represents an alkyl group which may be substituted, or a phenyl group)
and a 1-halogeno-2-deoxy-α-D-erythro-pentofuranose derivative represented by the general formula (III) 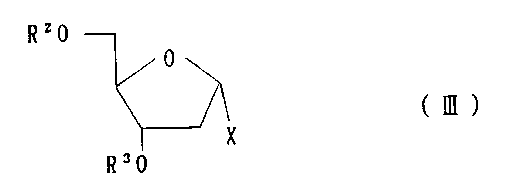 (wherein R² and R³ represent a general protective group for a hydroxyl group, and X represents a halogen atom)
are subjected to a condensation reaction to give a 1-(2-deoxy-β-D-erythro-pentofuranosyl)-5-trifluoromethyluracil derivative represented by the general formula (II) 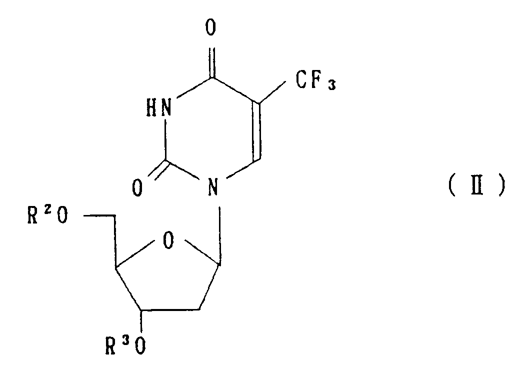 (wherein R² and R³ represent a general protective group for a hydroxyl group)
which is then subjected to a deprotection reaction to give 2'-deoxy-5-trifluoromethyl-β-uridine.

2. A process as claimed in claim 1, wherein chloroform is used as the solvent for the condensation reaction.

3. A process as claimed in either of claims 1 and 2, wherein said condensation reaction is carried out in the presence of a Lewis acid as a catalyst.

4. A process as claimed in claim 3, wherein said Lewis acid is zinc chloride.

5. A process as claimed in any of claims 1 to 4, wherein the molar ratio of compound (III) and compound (IV) in the condensation reaction is from 1:1 to 1:10.

6. A process as claimed in claim 5, wherein said molar ratio of compound (III) and compound (IV) in the condensation reaction is 1:4.

7. A process as claimed in any of claims 1 to 6, wherein compound (IV) is obtained by the silylation of 5-trifluoromethyluracil.

8. A process as claimed in any of claims 1 to 7, wherein either or both of the triorganosilyl groups of compound (IV) are trimethylsilyl, t-butyldimethylsilyl and phenyldimethylsilyl groups.

9. A process as claimed in any of claims 1 to 8, wherein either or both of R₂ and R₃ in compound (IV) are aralkyl, acyl, alkyloxycarbonyl and aryloxylcarbonyl groups.

10. A process as claimed in any of claims 1 to 9, wherein the deprotection reaction is achieved by hydrolysis, alkanolysis or ammonolysis.

## Patentansprüche

1. Verfahren zur Herstellung von 2'-Desoxy-5-trifluormethyl-β-uridin der Formel (I) 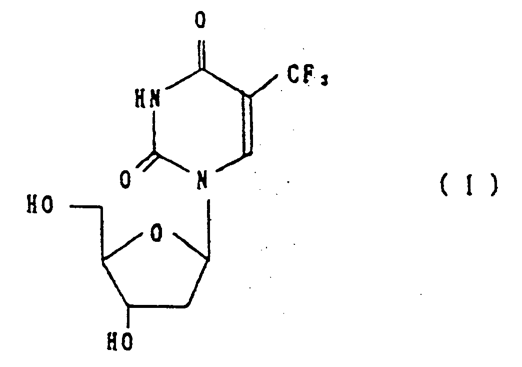 dadurch gekennzeichnet, daß
ein 5-Trifluormethyl-2,4-bis(triorganosilyloxy)pyrimidin der allgemeinen Formel (IV) 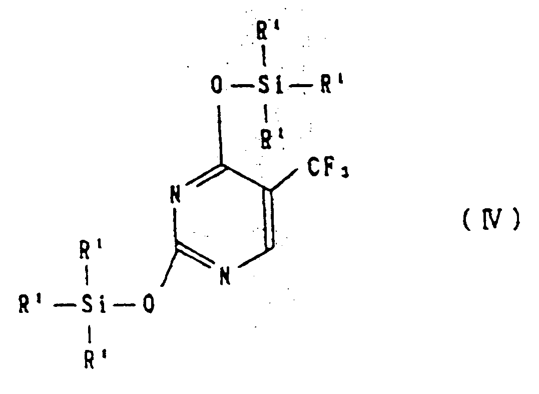 (worin jedes R', das gleich oder unterschiedlich sein kann, eine Alkylgruppe repräsentiert, die substituiert sein kann, oder eine Phenylgruppe)
und ein 1-Halogen-2-desoxy-α-D-erythro-pentofuranose-Derivat der allgemeinen Formel (III) 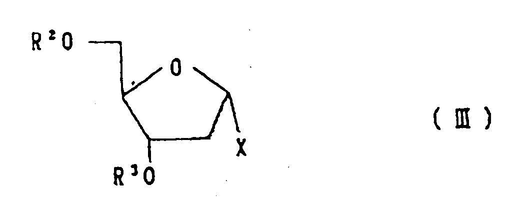 (worin R² und R³ eine allgemeine Schutzgruppe für eine Hydroxylgruppe repräsentieren, und X ein Halogenatom repräsentiert)
einer Kondensationsreaktion unterzogen werden, um ein 1-(2-Desoxy-β-D-erythro-pentofuranosyl)-5-trifluormethyluracil-Derivat der allgemeinen Formel (II) zu ergeben 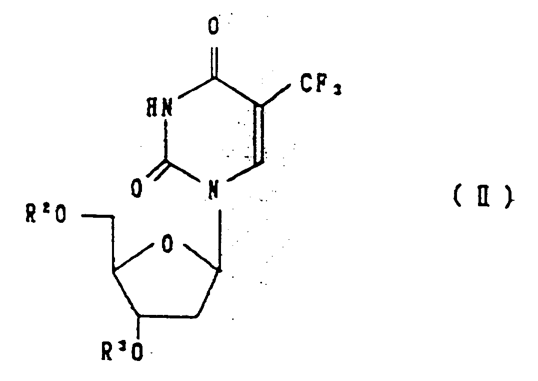 (worin R² und R³ eine allgemeine Schutzgruppe für eine Hydroxylgruppe repräsentieren),
die dann einer Reaktion zur Entfernung der Schutzgruppe unterzogen wird, um 2-Desoxy-5-trifluormethyl-β-uridin zu ergeben.

2. Verfahren nach Anspruch 1, worin Chloroform als Lösungsmittel für die Kondensationsreaktion verwendet wird.

3. Verfahren nach einem der Ansprüche 1 und 2, wobei die Kondensationsreaktion in Gegenwart einer Lewis-Säure als Katalysator durchgeführt wird.

4. Verfahren nach Anspruch 3, wobei die Lewis-Säure Zinkchlorid ist.

5. Verfahren nach einem der Ansprüche 1 bis 4, wobei das Molverhältnis der Verbindung (III) und der Verbindung (IV) in der Kondensationsreaktion von 1:1 bis 1:10 reicht.

6. Verfahren nach Anspruch 5, wobei das Molverhältnis der Verbindung (III) und der Verbindung (IV) in der Kondensationsreaktion 1:4 beträgt.

7. Verfahren nach einem der Ansprüche 1 bis 6, wobei die Verbindung (IV) durch die Silylierung von 5-Trifluormethyluracil erhalten wird.

8. Verfahren nach einem der Ansprüche 1 bis 7, wobei eine oder beide der Triorganosilylgruppen der Verbindung (IV) Trimethylsilyl-, t-Butyldimethylsilyl- und Phenyldimethylsilylgruppen sind.

9. Verfahren nach einem der Ansprüche 1 bis 8, worin einer oder beide Reste R₂ und R₃ in der Verbindung (IV) Aralkyl-, Acyl-, Alkyloxycarbonyl- und Aryloxycarbonylgruppen sind.

10. Verfahren nach einem der Ansprüche 1 bis 9, worin die Reaktion zur Entfernung der Schutzgruppe durch Hydrolyse, Alkanolyse oder Ammonolyse durchgeführt wird.

## Revendications

1. Procédé de préparation de la 2'-désoxy-5-trifluorométhyl-β-uridine représentée par la formule (I) 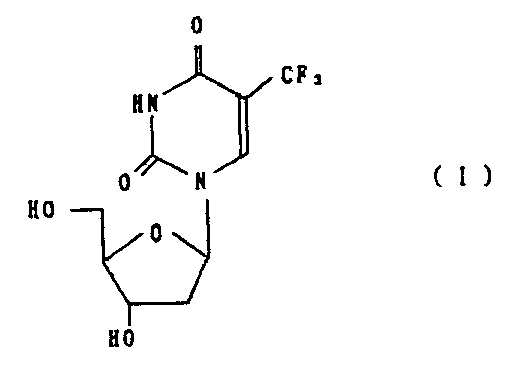 caractérisé en ce qu'une 5-trifluorométhyl-2,4-bis(triorganosilyloxy)pyrimidine représentée par la formule générale (IV) 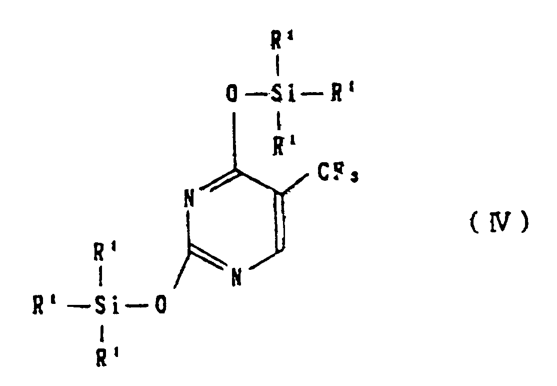 (dans laquelle les radicaux R¹, qui peuvent être identiques ou différents, représentent chacun un groupement alkyle qui peut être substitué ou un groupement phényle)
et un dérivé de 1-halogéno-2-désoxy-α-D-érythro-pentofuranose représenté par la formule générale (III) 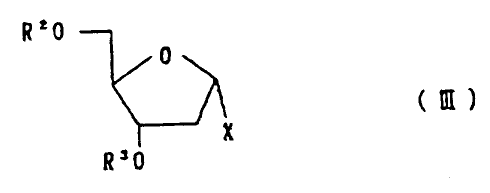 (dans laquelle R² et R³ représentent un groupement général de protection pour un groupement hydroxyle et X représente un atome d'halogène)
sont soumis à une réaction de condensation pour obtenir un dérivé du 1-(2-désoxy-β-D-érythro-pentofuranosyl)-5-trifluorométhyluracile représenté par la formule générale (II) 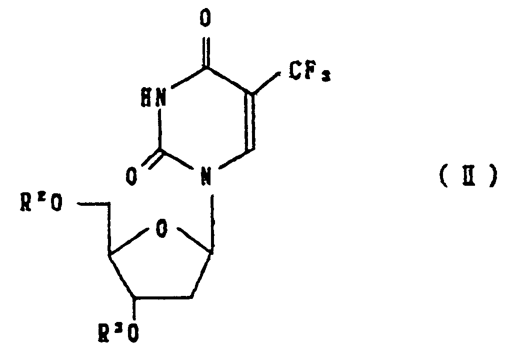 (dans laquelle R² et R³ représentent un groupement général de protection pour un groupement hydroxyle)
qui est ensuite soumis à une réaction de déprotection pour obtenir la 2'-désoxy-5-trifluorométhyl-β-uridine.

2. Procédé selon la revendication 1, dans lequel on utilise du chloroforme en tant que solvant pour la réaction de condensation.

3. Procédé selon la revendication 1 ou 2, dans lequel on met en oeuvre ladite réaction de condensation en présence d'un acide de Lewis en tant que catalyseur.

4. Procédé selon la revendication 3, dans lequel ledit acide de Lewis est le chlorure de zinc.

5. Procédé selon l'une quelconque des revendications 1 à 4, dans lequel le rapport molaire du composé (III) au composé (IV) dans la réaction de condensation est compris entre 1:1 et 1:10.

6. Procédé selon la revendication 5, dans lequel ledit rapport molaire du composé (III) au composé (IV) dans la réaction de condensation est égal à 1:4.

7. Procédé selon l'une quelconque des revendications 1 à 6, dans lequel on obtient le composé (IV) par la silylation du 5-trifluorométhyluracile.

8. Procédé selon l'une quelconque des revendications 1 à 7, dans lequel l'un des deux groupements triorganosilyle, ou les deux groupements, du composé (IV) sont des groupements du type triméthylsilyle, t-butyldiméthylsilyle et phényldiméthylsilyle.

9. Procédé selon l'une quelconque des revendications 1 à 8, dans lequel soit R₂, soit R₃, soit R₂ et R₃ du composé (IV) sont des groupements du type aralkyle, acyle, alkyloxycarbonyle et aryloxycarbonyle.

10. Procédé selon l'une quelconque des revendications 1 à 9, dans lequel la réaction de déprotection est obtenue par une hydrolyse, une alcanolyse ou une ammonolyse.
